(19) Europäisches Patentamt
European Patent Office
Office européen des brevets

(11) Veröffentlichungsnummer : **0 036 123**
**B1**

(12) # EUROPÄISCHE PATENTSCHRIFT

(45) Veröffentlichungstag der Patentschrift : **22.06.83**

(51) Int. Cl.[3] : **C 07 C 19/08**, C 07 C 17/00, B 01 J 27/06

(21) Anmeldenummer : **81101502.3**

(22) Anmeldetag : **03.03.81**

(54) **Verfahren zur Herstellung von hochreinen teilfluorierten Äthanen.**

(30) Priorität : **14.03.80 DE 3009760**

(43) Veröffentlichungstag der Anmeldung : **23.09.81 Patentblatt 81/38**

(45) Bekanntmachung des Hinweises auf die Patenterteilung : **22.06.83 Patentblatt 83/25**

(84) Benannte Vertragsstaaten : **BE DE FR GB IT NL**

(56) Entgegenhaltungen :
**DE A 2 712 732**
**DE A 2 837 515**

(73) Patentinhaber : **HOECHST AKTIENGESELLSCHAFT**
**Postfach 80 03 20**
**D-6230 Frankfurt/Main 80 (DE)**

(72) Erfinder : **von Halasz, Sigmar-Peter, Dr.**
**Feldbergstrasse 50**
**D-6233 Kelkheim (Taunus) (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

Verfahren zur Herstellung von hochreinen teilfluorierten Äthanen

Die vorliegende Erfindung betrifft ein Verfahren zur Herstellung von hochreinen teilfluorierten Äthanen durch Hydrofluorierung von fluorhaltigen Äthylenen.

Teilfluorierte Äthane der allgemeinen Formel

$$CF_3—CHXY,$$

wobei X = H oder F und Y = H, F, Cl, Br oder J bedeutet, sind aufgrund ihrer besonderen chemischen und physikalischen Eigenschaften für den Einsatz auf zahlreichen Anwendungsgebieten geeignet, z. B. als Additive in gasförmigen Analgetika und Anästhetika, als Wärmeaustauschmedien oder als Treibmittelkomponenten. Es besteht daher Interesse an einfachen und wirtschaftlichen Verfahren zur Herstellung von Vertretern dieser Verbindungsklasse.

Die genannten teilfluorierten Äthane sind an sich bekannt und können nach mehreren, meist labormäßigen, Verfahren hergestellt werden. Wenn fluorhaltige Äthylene der allgemeinen Formel $CF_2 = CXY$ hoher Reinheit in ausreichender Menge zur Verfügung stehen, bietet sich die Hydrofluorierung in der Gasphase in Gegenwart eines Feststoff-Katalysators an.

Die Addition von Fluorwasserstoff an fluorhaltige Äthylene in der Gasphase unter Bildung von teilfluorierten Äthanen gemäß der Gleichung

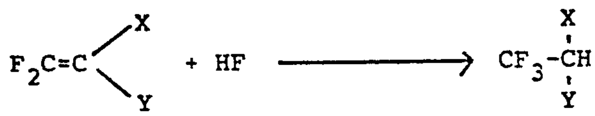

ist bereits für einige Verbindungen beschrieben worden. Jedoch sind bei allen bekannten Fluorierungsverfahren in der Gasphase relativ hohe Temperaturen erforderlich, meistens über 250 °C, teilweise sogar über 400 °C. Bei diesen Temperaturen kommt es jedoch zwangsläufig zu unerwünschten Nebenreaktionen. Beispielsweise kann bei Äthylenen, die Chlor, Brom- oder Jod-Atome enthalten, das Halogen durch Fluor substituiert werden. Auch im entstandenen teilfluorierten Äthan kann bei den genannten hohen Temperaturen das Halogen durch das Fluor des Fluorwasserstoffs verdrängt werden. Diese Reaktivität kann geradezu zur Herstellung von F-substituierten Verbindungen benutzt werden. Beispielsweise wird gemäß US-PS 2951 102 die Verbindung $CF_3CHClF$ aus $CF_2BrCHClF$ durch Reaktion mit HF bei 300 °C an einem Chromoxyfluorid-Katalysator bereitet.

In der DE-OS 28 37 515 wird die Hydrofluorierung von Trifluoräthylen bei Reaktionstemperaturen von 350 °C beschrieben. Verwendet wird dabei ein Chromoxyfluorid-Katalysator, der gemäß der britischen Patentschrift 1 307 224 hergestellt worden ist. Die Ausbeuten an 1,1,1,2-Tetrafluoräthan betragen 90 bis 95 % der Theorie. Die möglichen Verweilzeiten sind eng begrenzt und der Anteil an fluorierten Nebenprodukten ist hoch.

Ebenfalls an einem Chromoxyfluorid-Katalysator kann man gemäß US-PS 3 755 477 Chlortrifluoräthylen bei 320 °C zu einem Gemisch umsetzen, das unter anderem 2-Chlor-1,1,1,2-tetrafluoräthan (13 % der Theorie) enthält. Das gleiche Hydrofluorierungsverfahren wird gemäß kanadischer Patentschrift 849 024 bei 350 bis 400 °C durchgeführt ; dabei wird ein beträchtlicher Teil des Ausgangsprodukts nicht umgesetzt.

Bei eigenen Versuchen die bekannten Gasphasen-Verfahren zur Hydrofluorierung auf die genannten fluorhaltigen Äthylene zu übertragen, wurden folgende Nebenreaktionen beobachtet : Isomerisierungen, Disproportionierungen, Oligomerisierungen, Polymerisierungen, Fragmentierung der C-C-Bindungen sowie Substituenten, z. B. von Chlor oder Wasserstoff gegen Fluor. Diese Nebenreaktionen verringern die Ausbeuten und führen zu Nebenprodukten, die oft mit beträchtlichem Aufwand anschließend abgetrennt werden müssen. Andererseits sind die Reaktionstemperaturen bei den bekannten Verfahren unwirtschaftlich hoch. Damit wird auch in erheblichem Maße die Lebensdauer der eingesetzten Katalysatoren begrenzt, insbesondere bei Einsatz von Äthylenen, die Wasserstoff, Brom oder Jod enthalten und die daher geeignet sind, bei hohen Temperaturen den Katalysator zu vergiften.

Es bestand daher die Aufgabe, ein einfaches, ergiebiges und umweltfreundliches Verfahren zur Herstellung von hochreinen teilfluorierten Äthanen zu schaffen, das bei möglichst niedrigen Temperaturen durchführbar ist, mit hoher Selektivität und mit hohen Ausbeuten abläuft, bei dem also die bekannten Nebenreaktionen keine Rolle spielen und das leicht kontinuierlich angewandt werden kann.

Es wurde nun ein Verfahren zur Herstellung hochreiner fluorhaltiger Äthane der allgemeinen Formel $CF_3—CHXY$, wobei X = H oder F und Y = H, F, Cl, Br oder J bedeutet, gefunden, bei dem man fluorhaltige Äthylene der allgemeinen Formel $CF_2 = CXY$, in der X und Y die oben angegebene Bedeutung haben, mit mindestens der äquimolaren Menge Fluorwasserstoff in der Gasphase umsetzt. Das Verfahren ist dadurch gekennzeichnet, daß man bei Temperaturen von 20 bis 200 °C und in Gegenwart eines Chromoxyfluorid-Katalysators arbeitet, der mit Fluorwasserstoff aktiviert wurde.

Der noch mit Fluorwasserstoff zu aktivierende Chromoxyfluorid-Katalysator läßt sich z. B. durch

Umsetzung von Chromoxidhydraten mit Fluorwasserstoff (gemäß DE-AS 12 52 182) oder durch Erhitzen von wasserhaltigem Chromtrifluorid in Gegenwart von Sauerstoff (US-PS 2 745 886) herstellen.

Jene Katalysatoren, die aus Chromoxidhydrat durch Behandeln mit Fluorwasserstoff erhalten werden, erfordern eine Aktivierung mit Fluorwasserstoff nur dann, wenn sie erst nach längerer Zeit, z. B. nach einigen Monaten, für das erfindungsgemäße Verfahren eingesetzt werden. Falls die gleichen Katalysatoren jedoch sofort nach ihrer Herstellung benützt werden, so ist eine Aktivierung mit Fluorwasserstoff entbehrlich. Bei den aus Chromtrifluorid hergestellten Katalysatoren ist die Aktivierung mit Fluorwasserstoff jedoch stets erforderlich.

Die Aktivierung der Chromoxyfluorid-Katalysatoren erfolgt entweder für eine längere Zeit (mehr als 48 Stunden) mit Fluorwasserstoff im allgemeinen bei Temperaturen von 250 bis 300 °C oder noch besser für eine kürzere Zeit (ca. 5 bis 10 Stunden) mit einem Gasgemisch, das Fluorwasserstoff und elementares Fluor enthält. Dabei wird im allgemeinen elementares Fluor mit einem Inertgas und wasserfreiem Fluorwasserstoff verdünnt und in ein mit dem Chromoxidfluorid-Katalysator gefülltes auf Temperaturen von 150 bis 210 °C geheiztes Rohr eingeleitet. Die Aktivierungszeit hängt von der Vorgeschichte des Katalysators ab und beträgt im allgemeinen 5 bis 10 Stunden. Zweckmäßigerweise beträgt die Rate pro Liter Kontaktmaterial des eindosierten Fluors 0,1 bis 5,0 l/h, des Inertgases 1,0 bis 3,0 l/h und des Fluorwasserstoffs 10 bis 50 g/h. Selbstverständlich kann die Behandlung mit Fluorwasserstoff/Fluor nicht nur gleichzeitig sondern auch nacheinander erfolgen.

Die Behandlung eines Chromoxyfluorid-Katalysators mit einem Gemisch von Fluorwasserstoff und elementarem Fluor ist im wesentlichen aus der DE-OS 27 02 360 bekannt und dient dort zur Reaktivierung von Katalysatoren, die für Chlor-Fluor-Austausch-Reaktionen eingesetzt werden.

Das erfindungsgemäße Verfahren wird im allgemeinen nach Art üblicher katalytischer Gas-Feststoff-Reaktionen durchgeführt, indem man ein Gasgemisch, bestehend aus dem umzusetzenden fluorhaltigen Äthylen und Fluorwasserstoff, durch ein heizbares Reaktionsrohr leitet, das mit dem oben erwähnten aktivierten Chromoxyfluorid-Katalysator gefüllt ist. Das Reaktionsrohr wird vorzugsweise vertikal aufgestellt.

Das Reaktionsrohr besteht aus einem gegen Fluorwasserstoff und elementarem Fluor hinreichend resistenten Werkstoff, wie Nickel, Stahl, Kupfer oder Platin, oder ist mit einer geeigneten Innenauskleidung, z. B. aus Polytetrafluoräthylen oder einem anderen geeigneten hochfluorierten Polymeren ausgestattet. Die Reaktionsgase, die das Reaktionsrohr verlassen, werden durch eine Waschung mit Wasser oder durch eine Absorption in Türmen mit granuliertem Natriumfluorid von überschüssigem Fluorwasserstoff befreit. Das so vorgereinigte teilfluorierte Äthan wird in geeigneten Kühlfallen kondensiert.

Die Formgebung des eingesetzten Chromoxyfluorid-Katalysators ist nicht kritisch ; üblicherweise werden kugen-, würfel- oder zylinderförmige Körper verwendet mit einem Rauminhalt von 0,01 bis 10 ccm.

Der Einsatz der Verbindungen Trifluoräthylen, Tetrafluoräthylen, Chlortrifluoräthylen, Bromtrifluoräthylen oder Trifluorjodäthylen ist beim erfindungsgemäßen Verfahren bevorzugt. Dabei erhält man 1,1,1,2-Tetrafluoräthan, Pentafluoräthan, 2-Chlor-1,1,1,2-tetrafluoräthan, 1-Brom-1,2,2,2-tetrafluoräthan bzw. 1,2,2,2-Tetrafluor-1-jod-äthan in nahezu quantitativen Ausbeuten.

Zur Durchführung des erfindungsgemäßen Verfahrens werden die fluorhaltigen Äthylene im Gaszustand zunächst mit Fluorwasserstoff gemischt und anschließend in den mit dem Katalysator gefüllten Reaktor eingeleitet.

Für einige Ausgangsprodukte ist in der folgenden Tabelle der Siedepunkt aufgeführt :

Tabelle 1

| fluorhaltiges Äthylen | Formel | Siedepunkt/1 bar |
|---|---|---|
| 1,1-Difluoräthylen | $CF_2=CH_2$ | -82°C |
| Trifluoräthylen | $CF_2=CHF$ | -61°C |
| 2-Chlor-1,1-difluoräthylen | $CF_2=CHCl$ | -17,7°C |
| 1-Brom-2,2-difluoräthylen | $CF_2=CHBr$ | + 6,1°C |
| 2,2-Difluor-1-jod-äthylen | $CF_2=CHJ$ | + 34°C |
| Tetrafluoräthylen | $CF_2=CF_2$ | -76,3°C |
| Chlortrifluoräthylen | $CF_2=CFCl$ | -27,9°C |
| Bromtrifluoräthylen | $CF_2=CFBr$ | - 4,5°C |
| Trifluorjodäthylen | $CF_2=CFJ$ | +29°C |

Die fluorhaltigen Äthylene werden im allgemeinen in technischer Reinheit, zweckmäßigerweise

möglichst frei von Wasser verwendet. Diese Ausgangsprodukte sind nach an sich bekannten Verfahren in einfacher Weise herstellbar ; einige Vertreter stehen auch im technischen Maßstab zur Verfügung, wie z. B. Tetrafluoräthylen, Chlortrifluoräthylen oder 1,1-Difluoräthylen.

Bei Atmosphärendruck beträgt der Durchsatz an fluorhaltigen Äthylenen zweckmäßigerweise etwa 1 bis 90 l (ca. 0,04 bis 4 Mol) pro Liter Katalysator und Stunde. Bei höheren Drucken kann der Durchsatz an fluorhaltigen Äthylenen entsprechend höher liegen. Niedrigere Durchsätze sind möglich, aber unwirtschaftlich.

Das erfindungsgemäße Verfahren findet im allgemeinen bei Normaldruck statt, erlaubt jedoch auch die Anwendung von Über- oder Unterdruck in weiten Grenzen. So kann man bei Drucken unter 1 bar oder auch bei Überdruck von 1 bis 10 bar oder mehr, vorzugsweise bei 1 bis 3 bar, arbeiten. Insbesondere zur Erzielung höherer Raum-Zeit-Ausbeuten ist die Anwendung von Überdruck vorzuziehen.

Falls die Ausgangsverbindungen nicht wasserfrei sind, kann es zu Nebenreaktionen (Bildung sauerstoffhaltiger Produkte) kommen. Fluorwasserstoff wird im allgemeinen ohne Verdünnen zugegeben. Die Menge an gasförmig eindosiertem Fluorwasserstoff liegt im allgemeinen zwischen 0,8 und 250 g (bzw. 0,04 und 12,5 Mol) pro Liter Katalysator und Stunde. Sie soll der Menge des jeweils eingesetzten fluorhaltigen Äthylens mindestens äquivalent sein, vorzugsweise aber höher sein. Das Molverhältnis von fluorhaltigem Äthylen zu Fluorwasserstoff beträgt im allgemeinen 1 : 3 bis 1 : 1, vorzugsweise 1 : 2 bis 1 : 1,1, insbesondere 1 : 1,5 bis 1 : 1,1. Die insgesamt eingeleitete Menge an Fluorwasserstoff ist, sofern man nur einen Überschuß (bezogen auf eingesetztes fluorhaltiges Äthylen) verwendet, nicht kritisch. Ein Überschuß an Fluorwasserstoff ist insofern günstig, als dadurch ein quantitativer Umsatz des fluorhaltigen Äthylens erreicht wird. Auch größere Überschüsse an Fluorwasserstoff sind möglich ; jedoch nimmt dadurch der Aufwand bei der Aufarbeitung zu. Überschüssiger Fluorwasserstoff wird aus dem Reaktionsgas entweder durch Waschen mit Wasser oder verdünnter Natronlauge entfernt oder in Türmen mit granuliertem Natriumfluorid bei Raumtemperatur absorbiert. Da der Fluorwasserstoff nach der Absorption an Natriumfluorid wieder thermisch desorbiert und erneut eingesetzt werden kann, bleiben bei dieser Fahrweise die Verluste an Fluorwasserstoff sehr klein. Auch eine Aufarbeitung durch fraktionierte Destillation ist möglich.

Im allgemeinen wird die Umsetzung ohne Zusatz eines Inertgases durchgeführt. Eine Verdünnung, z. B. mit Stickstoff oder einem anderen Inertgas, ist zwar möglich, bringt aber nur eine minimale Erhöhung der Ausbeute mit sich.

Die Reaktionen werden bei Temperaturen von 20 bis 200 °C, vorteilhafterweise von 40 bis 190 °C, insbesondere von 60 bis 180 °C durchgeführt.

Die Verweilzeit von fluorhaltigem Äthylen, bzw. des daraus entstandenen fluorhaltigen Äthans im Reaktor ist nicht kritisch. Sie kann in beiden Fällen zwischen einigen Sekunden und einigen Minuten schwanken ; sie ist nach oben lediglich durch wirtschaftliche Erwägungen begrenzt. Es ist als besonderer Vorteil des erfindungsgemäßen Verfahrens anzusehen, daß die Verweilzeit in weiten Grenzen variiert werden kann, ohne daß die Zusammensetzung des Reaktionsprodukts dadurch beeinflußt wird.

Beim erfindungsgemäßen Verfahren ist eine kontinuierliche Fahrweise, d. h. kontinuierliches Einleiten der Ausgangsprodukte, kontinuierliche Rückführung des überschüssigen Fluorwasserstoffs sowie kontinuierliche Isolierung des gebildeten teilfluorierten Äthans ohne weiteres möglich. Besondere Vorteile der kontinuierlichen Fahrweise liegen in der hohen Ausnutzung der eingesetzten Ausgangsprodukte sowie in dem geringen Anfall an Abwasser und Abgas.

Der Umsatz der eingesetzten fluorhaltigen Äthylene liegt bei dem erfindungsgemäßen Verfahren im allgemeinen bei über 95 %, oft jedoch bei 99-100 %.

Aufgrund der hohen Selektivität des erfindungsgemäßen Verfahrens liegen die Ausbeuten an teilfluorierten Äthanen ebenfalls über 95 %, oft jedoch bei über 98 %. Somit fallen die Äthane in hoher Reinheit an. Als vorteilhaft erweist sich das erfindungsgemäße Verfahren vor allem darin, daß Nebenreaktionen nahezu vollständig unterdrückt werden und sich die Aufarbeitung dadurch als außerordentlich einfach erweist.

Die folgende Tabelle gibt die Siedepunkte der teilfluorierten Äthane wieder, die sich aus den fluorhaltigen Äthylenen der Tabelle 1 herstellen lassen.

Tabelle 2 : Äthane

| Bezeichnung | Formel | Siedepunkt/1 bar |
|---|---|---|
| 1,1,1-Trifluoräthan | $CF_3-CH_3$ | -47,8°C |
| 1,1,1,2-Tetrafluoräthan | $CF_3-CH_2F$ | -26,5°C |
| 2-Chlor-1,1,1-trifluoräthan | $CF_3-CH_2Cl$ | + 6,1°C |
| 1-Brom-2,2,2-trifluoräthan | $CF_3-CH_2Br$ | +26°C |
| 2,2,2-Trifluor-1-jod-äthan | $CF_3-CH_2J$ | +55°C |
| Pentafluoräthan | $CF_3-CHF_2$ | -48,5°C |

Tabelle 2 (Fortsetzung)

| Bezeichnung | Formel | Siedepunkt/1 bar |
|---|---|---|
| 2-Chlor-1,1,1,2-tetrafluor-äthan | $CF_3$-CHClF | -12°C |
| 1-Brom-1,2,2,2-tetrafluor-äthan | $CF_3$-CHBrF | + 7°C |
| 1,2,2,2-Tetrafluor-1-jod-äthan | $CF_3$-CHFJ | +39°C |

Aus der DE-OS 27 12 732 ist es bekannt, daß Perfluorpropylen mit Fluorwasserstoff bei Temperaturen von 100 bis 350 °C in Gegenwart von Chromoxyfluorid-Katalysatoren reagiert. Dabei sind die Ausbeuten an 2H-Heptafluorpropan bei Reaktionstemperaturen unter 200 °C nur mäßig. Sie betragen z. B. bei 160 bis 170 °C nur 82 % der Theorie. Außerdem geht aus dieser Literaturstelle die Bedeutung der Aktivierung des Katalysators mit Fluorwasserstoff nicht hervor.

Es ist überraschend, daß in Gegenwart der mit HF aktivierten Chromoxyfluorid-Katalysatoren die Hydrofluorierung von fluorhaltigen Äthylenen mit hohen Umsätzen, oft quantitativ, und mit äußerst hoher Selektivität verläuft. Insbesondere überrascht, daß die Reaktionstemperaturen des erfindungsgemäßen Verfahrens im Vergleich zum Stand der Technik unerwartet niedrig liegen.

Das erfindungsgemäße Verfahren stellt einen erheblichen technischen Fortschritt dar, da es auf der Basis der entweder technisch erhältlichen oder über andere technische Zwischenprodukte leicht zugänglichen fluorhaltigen Äthylene unter optimaler Ausnutzung der Ausgangsstoffe, ohne wesentliche Bildung von üblicherweise anfallenden Isomerisierungs-Disproportionierungs-, Oligomerisierungs-, Polymerisierungs-, Fragmentierungs- oder Substitutionsprodukten, die Herstellung von teilfluorierten Äthanen in Ausbeuten von über 95 %, teilweise bis 99 % ermöglicht. Damit verbunden ist eine einfache Aufarbeitung und ein leicht erreichbarer hoher Reinheitsgrad der Endprodukte. Weiterhin ist das erfindungsgemäße Verfahren aufgrund der niedrigen Reaktionstemperaturen und den damit verbundenen hohen Standzeiten der eingesetzten Katalysatoren von großem Interesse.

Das Verfahren wird durch folgende Beispiele näher erläutert :

## Beispiel 1

Die Aktivierung der Chromoxyfluorid-Katalysatoren mit Fluorwasserstoff oder mit einem Gasgemisch aus Fluorwasserstoff und elementarem Fluor und die anschließende Hydrofluorierung der fluorhaltigen Äthylene wurden in derselben Versuchsanlage durchgeführt.

Diese besteht aus einem senkrecht stehenden Nickelrohr mit einer Länge von 150 cm und einem Innendurchmesser von 5 cm.

Von außen wird der Reaktor mit einem Heizmantel beheizt ; im Inneren des Reaktorrohrs verläuft axial ein VA-Stahlrohr mit einem Außendurchmesser von 0,6 cm, einem Innendurchmesser von 0,4 cm und einer Länge von 145 cm, durch das ein Thermoelement zur Messung der Innentemperatur in jeder gewünschten Reaktorhöhe eingeführt werden kann. Durch ein im Inneren des Reaktorrohres angebrachtes Siebteil wird das gekörnte Katalysatormaterial in der gewünschten Rohrhöhe gehalten. Am unteren Ende des Reaktors befindet sich ein getrennt beheizbares Verdampfergefäß aus Nickel, in das die Rohrleitungen für gasförmigen Fluorwasserstoff, verdünntes oder unverdünntes Fluorgas und für das gasförmige fluorhaltige Äthylen (in Beispiel 1 : 1,1-Difluoräthylen) hineinführen. Das Verdampfergefäß wird auf einer Temperatur zwischen dem Siedepunkt von Fluorwasserstoff (+ 20 °C) und der Reaktortemperatur gehalten. Vom oberen Ende des Nickelreaktors führt eine Rohrleitung in ein mit Wasser gefülltes Waschvorlagegefäß, in dem bei den Hydrofluorierungen nicht umgesetzter Fluorwasserstoff aufgefangen und probeweise titriert wird. Bei den Aktivierungen mit elementarem Fluor wird zwischen Reaktorausgang und Waschgefäß eine Vorlage mit Hexafluorpropentrimeren geschaltet. Damit kann überschüssiges Fluor gefahrlos absorbiert werden (vgl. DE-OS 23 32 097) ; zusätzliche Fluortests werden bei den Aktivierungen mit Kalium-Jodid-Papier vorgenommen.

Elementares Fluor ($F_2$) wird einer handelsüblichen Stahlflasche entnommen, mit einem zuvor geeichten Differenzdruck-Strömungsmesser gemessen und nach Verdünnen mit Stickstoff in den Reaktor geleitet. Vor dem Fluorströmungsmesser ist ein gleichzeitig als Sicherheitsventil dienendes Steigrohrmanometer zur Beobachtung des sich einstellenden Staudrucks angebracht. Die Staudruckmesser sind mit perfluorierten Polyätherölen gefüllt.

Fluorwasserstoff (HF) wird mit der über 99 %igen handelsüblichen Reinheit eingesetzt und mit einer prinzipiell gleichartigen Meßanordnung, wie beim Fluor beschrieben, dosiert. Zusätzlich sind Heizstrahler angebracht, um eine Kondensation von HF in den Rohrleitungen bis zum Verdampfer zu verhindern.

Im Reaktorrohr werden 600 ml (Schüttvolumen) eines Chromoxyfluorid-Katalysators vorgelegt, der durch Fluorierung von Chromoxidhydratgrün mit Fluorwasserstoff gemäß DE-AS 1 252 182 hergestellt worden war. Zur Aktivierung wird der vorgelegte Katalysator bei einer Innentemperatur von 190 °C für 10 Stunden mit einem Gasgemisch, bestehend aus 0,35 l/h Fluor, 0,5 l/h Stickstoff und 15 g/h Fluorwasserstoff, behandelt ; anschließend wird bei derselben Innentemperatur für 5 Stunden mit 15 g/h Fluorwasserstoff gespült.

Das 1,1-Difluoräthylen wird einer handelsüblichen Stahlflasche entnommen. Zur Hydrofluorierung werden bei Reaktortemperaturen von 80 °C (zu Beginn) bis 98 °C (nach Eintritt der exothermen Additionsreaktion) über den aktivierten Chromoxyfluoridkatalysator innerhalb von 6 Stunden insgesamt 420 g (6,56 Mol) $CF_2 = CH_2$ und 237 g (11,85 Mol) HF, entsprechend einem Molverhältnis von $CF_2 = CH_2$ : HF wie 1 : 1,81, eingeleitet.

Im Waschwasser werden 5,23 Mol HF aufgefangen. Das gasförmige Reaktionsprodukt wird zur Trocknung durch einen $CaCl_2$-Trockenturm geführt und anschließend in einer mit festem Kohlendioxid gekühlten Intensivfalle kondensiert. Die gaschromatographische Messung dieses Rohproduktes an einer PORAPAK®-Säule ergibt folgende Zusammensetzung :

| | |
|---|---|
| $CF_3—CH_3$ | 99,6 % |
| $CF_2 = CH_2$ | 0,3 % |
| $CHF_3$ | < 0,05 % |
| $CH_2F_2$ | < 0,05 % |
| $CHF_2—CH_3$ | < 0,05 % |

Das kondensierte Rohprodukt wiegt 537 g. Damit beträgt die Ausbeute an $CF_3—CH_3$ 97,3 % d. Th., bezogen auf umgesetztes $CF_2 = CH_2$. Eine weitere Identifizierung von 1,1,1-Trifluoräthan erfolgt durch Infrarot-, $^{19}F$- und $^1H$-NMR-Spektren. Der Siedepunkt des Produktes liegt bei − 48 bis − 47 °C.

## Beispiel 2

In der Versuchsanordnung aus Beispiel (1) werden zur Hydrofluorierung von Trifluoräthylen bei Temperaturen von 124 bis 143 °C über den im Beispiel (1) benutzten Chromoxyfluorid-Katalysator innerhalb von 3 Stunden insgesamt 235 g (2,87 Mol) $CF_2 = CHF$, das durch Dehalogenierung von $CBrF_2—CHClF$ hergestellt worden ist, und 83 g (4,15 Mol) HF, entsprechend einem Molverhältnis von $CF_2 = CHF$ : HF wie 1 : 1,45, geleitet.

In Waschwasser werden 1,25 Mol HF titriert. Die gaschromatographische Aufnahme des in der Falle aufgefangenen Rohproduktes ergibt folgende Werte :

| | |
|---|---|
| $CF_3—CH_2F$ | 99,7 % |
| $CF_2 = CHF$ | < 0,05 % |
| $CHF_3$ | < 0,05 % |
| $CF_3—CH_3$ | < 0,05 % |

Das Kondensat wiegt 286,5 g ; damit beträgt die Ausbeute an 1,1,1,2-Tetrafluoräthan 97,6 % d. Th., bezogen auf umgesetztes $CF_2 = CHF$. Die Charakterisierung von 1,1,1,2-Tetrafluoräthan erfolgt durch IR-, $^{19}F$- und $^1H$-NMR-Messungen ; der Siedepunkt liegt bei − 27 °C bis − 26 °C.

## Beispiel 3

In der Versuchsanordnung aus Beispiel (1) werden 600 ml (Schüttvolumen) eines Chromoxyfluorid-Katalysators vorgelegt, der gemäß DE-AS 1 252 182 hergestellt worden ist. Eine Aktivierung des Katalysatormaterials erfolgt in diesem Falle zunächst nur mit Fluorwasserstoff ; dabei werden bei Temperaturen von 330 °C bis 350 °C innerhalb von 72 Stunden insgesamt 1 080 g HF bei einer Rate von 15 g/h über den vorgelegten Katalysator geleitet.

Über den in der beschriebenen Weise vorbereiteten Katalysator werden bei Temperaturen von 125 bis 142 °C innerhalb von 3 Stunden insgesamt 243 g (2,96 Mol) $CF_2 = CHF$ und 86 g (4,30 Mol) HF, entsprechend einem Molverhältnis von $CF_2 = CHF$ : HF wie 1 : 1,45 geleitet.

Im Waschwasser werden 1,45 Mol HF aufgefangen. Das kondensierte Rohprodukt setzt sich gemäß einer gas chromatographischen Messung wie folgt zusammen :

| | |
|---|---|
| $CF_3—CH_2F$ | 95,8 % |
| $CF_2 = CHF$ | 4,0 % |
| $CHF_3$ | < 0,05 % |
| $CF_3CH_3$ | < 0,05 % |

Das Rohprodukt hat ein Gewicht von 293,5 g ; bezogen auf umgesetztes $CF_2 = CHF$ beträgt damit die Ausbeute an $CF_3—CH_2F$ 97 % d. Th., der Umsatz an $CF_2 = CHF$ erreicht jedoch nur ca. 96 %.

## Beispiel 4

In der Versuchsanordnung aus Beispiel (1) wird der in Beispiel (3) verwendete Katalysator zur Aktivierung — wie in Beispiel (1) beschrieben — bei einer Innentemperatur von 190 bis 195 °C für 10 Stunden mit einem Gasgemisch, bestehend aus 0,35 l/h Fluor, 0,05 l/h $N_2$ und 15 g/h HF, vorbehandelt. Anschließend wird bei 190 °C für 5 Stunden mit 15 g/h HF gespült.

Über den in der beschriebenen Weise vorbereiteten Chromoxyfluorid-Katalysator wird bei einer Innentemperatur von 75 °C (zu Anfang) bis 96 °C (durch Wärmetönung der Umsetzung bedingt) innerhalb von 26 Stunden ein Gasgemisch, bestehend aus insgesamt 1 698 g (20,7 Mol) $CF_2 = CHF$ und 666 g (33,3 Mol) HF bei einem Molverhältnis von $CF_2 = CHF$ : HF wie 1 : 1,61, geführt.

Im Waschwasser werden 12,55 Mol HF gefunden. Die gaschromatographischen Messungen ergeben für das aufgefangene Rohprodukt folgende Zusammensetzung :

| | |
|---|---|
| $CF_3—CH_2F$ | 99,8 % |
| $CF_2 = CHF$ | < 0,05 % |
| $CHF_3$ | < 0,05 % |
| $CF_3—CH_3$ | < 0,05 % |

Das Kondensat wiegt 2 088 g ; damit beträgt die Ausbeute an $CF_3—CH_2F$ ca. 99 % d. Th., bezogen auf umgesetztes $CF_2 = CHF$, das in diesem Beispiel bei stark gemäßigten Temperaturen quantitativ umgesetzt werden konnte.

## Beispiel 5

In der Versuchsapparatur aus Beispiel (1) wird über den im Beispiel (3) verwendeten Chromoxyfluorid-Katalysator bei einer Innentemperatur von 120 bis 131 °C innerhalb von 6,0 Stunden ein Gasgemisch, bestehend aus insgesamt 510 g (5,18 Mol) 2-Chlor-1,1-difluoräthylen, das durch Dechlorierung von 1,1,2-Trichlor-2,2-difluoräthan hergestellt worden ist, und 153 g (7,65 Mol) HF bei einem Molverhältnis von $CF_2 = CHCl$ : HF wie 1 : 1,48, geleitet.

Im Waschwasser werden 2,33 Mol HF und 0,04 Mol HCl titriert.

Gemäß den gaschromatographischen Untersuchungen besteht das Rohprodukt aus folgenden Bestandteilen :

| | |
|---|---|
| $CF_3—CH_2Cl$ | 99,0 % |
| $CF_2 = CHCl$ | < 0,05 % |
| $CF_3—CH_2F$ | 0,8 % |
| $CF_3—CH_3$ | < 0,05 % |

Das aufgefangene Rohprodukt hat ein Gewicht von 603 g ; damit beträgt die erhaltene Ausbeute an 2-Chlor-1,1,1-trifluoräthan 97,2 % d. Th., bezogen auf umgesetztes $CF_2 = CHCl$. Das gewonnene Äthan $CF_3—CH_2Cl$ wurde zusätzlich durch Aufnahme von IR-, $^{19}F$- und H-NMR-Spektren sowie durch Bestimmung des Siedepunktes von + 6 °C bis + 6,5 °C charakterisiert.

## Beispiel 6

Zur Hydrofluorierung von Tetrafluoräthylen, das in der für Polymerisationen hohen erforderlichen Reinheit eingesetzt wird, wird in der im Beispiel (1) verwendeten Apparatur 600 ml (Schüttvolumen) Chromoxyfluorid-Katalysator mit einem Gasgemisch bestehend aus $F_2$, HF und $N_2$ wie in Beispiel (1) beschrieben, aktiviert.

Über den in dieser Weise aktivierten Katalysator werden bei einer Innentemperatur von 165 bis 179 °C innerhalb von 5,0 Stunden insgesamt 370 g (3,70 Mol) $CF_2 = CF_2$ und 170 g (8,5 Mol) HF, entsprechend einem Molverhältnis von $CF_2 = CF_2$ : HF wie 1 : 2,3, geführt.

Im Waschwasser werden 4,95 Mol HF aufgefangen. Die gaschromatographische Analyse ergibt für das erhaltene Rohprodukt folgende Werte :

| | |
|---|---|
| $CF_3—CHF_2$ | 92,5 % |
| $CF_2 = CF_2$ | 6,3 % |
| $CHF_3$ | 0,2 % |
| $CF_3—CF_3$ | 0,25 % |
| cyclo—$C_4F_8$ | ~ 1 % |

Das erhaltene Kondensat wiegt 417 g ; damit liegt die Ausbeute an Pentafluoräthan bei ca. 93 % d. Th., bezogen auf umgesetzes $CF_2 = CF_2$. Pentafluoräthan $CF_3—CHF_2$ wurde weiterhin durch IR- und NMR-Messungen charakterisiert. Eine nachfolgende Tieftemperaturdestillation ergab eine Fraktion von 372 g im Siedebereich von − 49 bis − 47,5 °C/Normaldruck, bestehend aus Pentafluoräthan zu über 99 %.

## Vergleichsbeispiel 1

In der Versuchsanordnung aus Beispiel (1), gefüllt mit dem Chromoxyfluorid-Katalysator aus Beispiel 6, werden bei einer Temperatur von 280 °C bis 288 °C innerhalb von 3,0 h insgesamt 202,5 g (2,03 Mol) $CF_2 = CF_2$ und 113 g (5,65 Mol) HF, entsprechend einem Molverhältnis von $CF_2 = CF_2 : HF$ wie 1 : 2,8, umgesetzt.

Im Waschwasser werden 4,05 Mol HF festgestellt. Aus der gaschromatographischen Messung des aufgefangenen Rohproduktes geht folgendes hervor :

| | |
|---|---|
| $CF_3—CHF_2$ | 83,5 % |
| $CF_2 = CF_2$ | < 0,05 % |
| $CF_3—CF_3$ | 3,5 % |
| cyclo—$C_4F_8$ | 3,3 % |
| $C_6F_{12}$ | 8,5 % |

Weiterhin werden auf dem Katalysatormaterial weiße, feste Rückstände, bestehend aus Polytetrafluoräthylen, beobachtet. Bei einem Gewicht von 184 g für das Kondensat (Rohprodukt) beträgt die Ausbeute an Pentafluoräthan in diesem Vergleichsbeispiel nur ca. 63 % d. Th., bezogen auf umgesetztes $CF_2 = CF_2$. Eine Erhöhung der Reaktortemperatur führt zwar zu einem quantitativen Umsatz an Äthylen, verursacht aber einen höheren Anteil an Nebenprodukten.

## Beispiel 7

Zur Hydrofluorierung von Chlortrifluoräthylen, das als technisches Zwischenprodukt in hoher Reinheit leicht erhältlich ist, wird in der Versuchsapparatur aus Beispiel (1) der im Beispiel (3) verwendete Chromoxyfluorid-Katalysator vorgelegt.

Bei einer Innentemperatur von 170 bis 178 °C werden innerhalb von 6,5 Stunden insgesamt 605 g (5,19 Mol) $CF_2 = CFCl$ und 152 g (7,6 Mol) HF, entsprechend einem Molverhältnis von $CF_2 = CFCl : HF$ wie 1 : 1,46, im Reaktorrohr umgesetzt.

Im Waschwasser werden 2,48 Mol HF und 0,03 Mol HCl titriert. Die Zusammensetzung des Rohkondensats wird anhand einer GC-Messung festgestellt :

| | |
|---|---|
| $CF_3—CHClF$ | 95,6 % |
| $CF_2 = CFCl$ | 3,0 % |
| $CF_3—CHCl_2$ | 0,4 % |
| $CF_3—CHF_2$ | 0,8 % |
| $CF_3—CH_2Cl$ | < 0,1 % |
| $CF_3—CClF_2$ | < 0,1 % |

Es wurden keine Polymerisate beobachtet. Das aufgefangene Rohprodukt wiegt 669 g ; damit beträgt die Ausbeute an $CF_3—CHClF$ 93,1 % d. Th., bezogen auf umgesetztes $CF_2 = CFCl$. Die weitere Charakterisierung erfolgt durch IR- und $^{19}F$- sowie $^1H$-NMR-Messungen. Eine nachfolgende Tieftemperaturdestillation ergibt bei − 12,5 bis − 11 °C eine Fraktion mit einem Gewicht von 612 g reinem 2-Chlor-1,1,1,2-tetrafluoräthan.

## Vergleichsbeispiel 2

In der Versuchsapparatur aus Beispiel (1), gefüllt mit dem Katalysator aus Beispiel (3), werden bei einer Innentemperatur von 270 bis 280 °C innerhalb von 4 Stunden insgesamt 354 g (3,04 Mol) $CF_2 = CFCl$ und 92 g (4,6 Mol) HF, entsprechend einem Molverhältnis von $CF_2 = CFCl : HF$ wie 1 : 1,51 durchgeleitet.

Im Waschwasser werden 1,19 Mol HF und 0,33 Mol HCl aufgefangen. Das Rohprodukt setzt sich wie folgt zusammen gemäß GC-Analyse :

| | | |
|---|---|---|
| | $CF_3—CHClF$ | 20,7 % |
| | $CF_2 = CFCl$ | < 0,05 % |
| (A) | $CF_3—CHCl_2$ | 29,6 % |
| (B) | $CF_3—CHF_2$ | 41,0 % |
| (C) | $CF_3—CH_2Cl$ | 4,5 % |
| (D) | $CF_3—CClF_2$ | 4,2 % |

Die Entstehung der Produkte (A) und (B) bzw. (C) und (D) läßt sich durch die verschiedenen Möglichkeiten der Disproportionierung von gebildetem $CF_3—CHClF$ erklären ; der höhere Anteil an Produkt (B), Pentafluoräthan, entsteht durch Chlor-Fluor-Austausch im $CF_3—CHClF$. Dieses Vergleichs-

beispiel zeigt deutlich den bestimmenden Einfluß der Reaktortemperatur auf die Zusammensetzung der Reaktionsprodukte.

Beispiel 8

Zur Hydrofluorierung von Bromtrifluoräthylen wird in der Versuchsapparatur aus Beispiel (1), gefüllt mit einem Katalysator wie in Beispiel (1) beschrieben, bei einer Temperatur von 165 bis 176 °C innerhalb von 4 Stunden ein Gasgemisch, bestehend aus insgesamt 334 g (2,07 Mol) $CF_2 = CFBr$ und 110 g (5,50 Mol) HF (Molverhältnis von $CF_2 = CFBr$ : HF wie 1 : 2,66) umgesetzt.

Im Waschwasser werden 3,44 Mol HF aufgefangen. Die GC-Analyse des Rohproduktes ergibt :

| | |
|---|---|
| $CF_3$—CHBrF | 94,5 % |
| $CF_2 = CFBr$ | 3,0 % |
| $CF_2Br$—$CHF_2$ | 1,1 % |
| $CF_3$—$CHF_2$ | < 1,0 % |

In der Falle sammelten sich 356 g Rohprodukt. Die Ausbeute an $CF_3$—CHBrF, liegt bei ca. 93 % d. Th., bezogen auf umgesetztes $CF_2 = CFBr$. Das gereinigte Rohprodukt wurde durch IR- und NMR-Messungen sowie durch Bestimmung des Siedepunkts von + 7 bis 7,5 °C charakterisiert.

Beispiel 9

Zur Hydrofluorierung von Trifluorjodäthylen, das z. B. durch Dehydrochlorierung von 2-Chlor-1,2,2-trifluor-1-jod-äthan nach bekannten Methoden hergestellt werden kann, wird die Versuchsapparatur aus Beispiel (1), gefüllt mit dem Katalysator aus Beispiel (1), benutzt. Durch den Reaktor werden bei einer Temperatur von 140 bis 146 °C innerhalb von 4,5 h insgesamt 249 g (1,20 Mol) $CF_2 = CFJ$ und 46 g (2,3 Mol) HF (entsprechend einem Molverhältnis von $CF_2 = CFJ$ : HF wie 1 : 1,9) geleitet.

Im Waschwasser werden 1,03 Mol HF titriert. Die GC-Analyse des Rohkondensats ergibt folgende Zusammensetzung :

| | |
|---|---|
| $CF_3$—CHFJ | 93,5 % |
| $CF_2 = CFJ$ | 2,5 % |
| $CF_3$—$CHF_2$ | 1,1 % |
| $CF_2J$—$CHF_2$ | 1,0 % |
| $CF_2J$—$CF_2J$ | < 1 % |

Das aufgefangene Rohprodukt wiegt 268 g ; damit beträgt die erhaltene Ausbeute an $CF_3$—CHFJ ca. 94 % d. Th., bezogen auf umgesetztes $CF_2 = CFJ$. Das erhaltene Hauptprodukt wurde weiterhin durch IR-, $^{19}F$- und $^{1}H$-NMR-Messungen charakterisiert. Der Siedepunkt des Äthans liegt bei + 39 °C/1 bar.

## Ansprüche

1. Verfahren zur Herstellung hochreiner fluorhaltiger Äthane der allgemeinen Formel $CF_3$—CHXY, wobei X = H oder F und Y = H, F, Cl, Br oder Jod bedeutet, aus fluorhaltigen Äthylenen der allgemeinen Formel $CF_2 = CXY$, in der X und Y die oben angegebene Bedeutung haben, mit mindestens der äquimolaren Menge Fluorwasserstoff in der Gasphase, dadurch gekennzeichnet, daß man bei Temperaturen von 20-200 °C und in Gegenwart eines Chromoxyfluorid-Katalysators arbeitet, der mit Fluorwasserstoff aktiviert wurde.
2. Verfahren nach Anspruch 1, dadurch gekennzeichnet, daß der Chromoxyfluorid-Katalysator zusätzlich mit elementarem Fluor aktiviert wurde.
3. Verfahren nach Anspruch 1, dadurch gekennzeichnet, daß der Chromoxyfluorid-Katalysator mit einem elementares Fluor und Fluorwasserstoff enthaltenden Gasgemisch aktiviert wurde.
4. Verfahren nach einem der Ansprüche 1 bis 3, dadurch gekennzeichnet, daß man bei Reaktionstemperaturen von 40°-190 °C, insbesondere 60-180 °C arbeitet.
5. Verfahren nach einem der Ansprüche 1 bis 4, dadurch gekennzeichnet, daß das Molverhältnis fluorhaltiges Äthylen zu Fluorwasserstoff 1 : 1 bis 1 : 3 beträgt.

## Claims

1. A process for the preparation of very pure fluorine-containing ethanes of the general formula $CF_3$—CHXY, in which X denotes H or F and Y denotes H, F, Cl, Br or iodine, from fluorine-containing ethylenes of the general formula $CF_2 = CXY$, in which X and Y have the meaning indicated above, with at least the equimolar amount of hydrogen fluoride, in the gas phase, which process comprises carrying out

the reaction at temperatures of 20-200 °C and in the presence of a chromium oxyfluoride catalyst which has been activated with hydrogen fluoride.

2. A process as claimed in claim 1, wherein the chromium oxyfluoride catalyst has additionally been activated with elementary fluorine.

3. A process as claimed in claim 1, wherein the chromium oxyfluoride catalyst has been activated with a gas mixture containing elementary fluorine and hydrogen fluoride.

4. A process as claimed in any of claims 1 to 3, which comprises carrying out the reaction at reaction temperatures of 40°-190 °C, in particular 60-180 °C.

5. A process as claimed in any of claims 1 to 4, wherein the molar ratio of fluorine-containing ethylene to hydrogen fluoride is 1 : 1 to 1 : 3.

**Revendications**

1. Procédé de préparation d'éthanes fluorés très purs qui répondent à la formule générale $CF_3$—CHXY dans laquelle X représente H ou F, et Y représente H, F, Cl, Br ou I, à partir d'éthylènes fluorés répondant à la formule générale $CF_2$ = CXY dans laquelle X et Y ont les significations qui viennent d'être données, avec une quantité au moins équimolaire de fluorure d'hydrogène, en phase gazeuse, procédé caractérisé en ce qu'on opère à des températures de 20 à 200 °C et en présence d'un catalyseur à base d'oxyfluorure de chrome qui a été activé par du fluorure d'hydrogène.

2. Procédé selon la revendication 1 caractérisé en ce que le catalyseur à l'oxyfluorure de chrome a été activé en outre par du fluor élémentaire.

3. Procédé selon la revendication 1 caractérisé en ce que le catalyseur à l'oxyfluorure de chrome a été activé par un mélange gazeux contenant du fluor élémentaire et du fluorure d'hydrogène.

4. Procédé selon l'une quelconque des revendications 1 à 3, caractérisé en ce qu'on opère à des températures réactionnelles de 40 à 190 °C, plus particulièrement de 60 à 180 °C.

5. Procédé selon l'une quelconque des revendications 1 à 4, caractérisé en ce que le rapport molaire de l'éthylène fluoré au fluorure d'hydrogène est compris entre 1 : 1 et 1 : 3.